**Europäisches Patentamt**

**European Patent Office**

**Office européen des brevets**

(19)

(11) Veröffentlichungsnummer : **0 153 562**
**B1**

(12) # EUROPÄISCHE PATENTSCHRIFT

(45) Veröffentlichungstag der Patentschrift :
12.11.86

(21) Anmeldenummer : 85100291.5

(22) Anmeldetag : 14.01.85

(51) Int. Cl.⁴ : **C 07 C143/24**, C 07 C139/00

(54) **Verfahren zur Herstellung von Diphenyl-4,4'-disulfonsäure.**

(30) Priorität : 26.01.84 DE 3402622

(43) Veröffentlichungstag der Anmeldung :
04.09.85 Patentblatt 85/36

(45) Bekanntmachung des Hinweises auf die Patenterteilung : 12.11.86 Patentblatt 86/46

(84) Benannte Vertragsstaaten :
CH DE FR GB IT LI

(56) Entgegenhaltungen :
DE-A- 1 804 693
DE-A- 2 723 333
DE-A- 3 226 392
US-A- 3 427 342
US-A- 4 226 796

(73) Patentinhaber : BAYER AG
Konzernverwaltung RP Patentabteilung
D-5090 Leverkusen 1 Bayerwerk (DE)

(72) Erfinder : Blank, Heinz Ulrich, Dr.
Am Geusfelde 35
D-5060 Odenthal (DE)
Erfinder : Köhler, Wilfried, Dr.
Gustav-Freytag-Strasse 2
D-5090 Leverkusen 1 (DE)
Erfinder : Schnegg, Peter, Dr.
Heidberger Strasse 44
D-5068 Odenthal (DE)

## Beschreibung

Die vorliegende Erfindung betrifft ein Verfahren zur Herstellung von Diphenyl-4,4'-disulfonsäure durch Sulfonierung von Diphenyl mit Schwefeltrioxid.

Diphenyl-4,4'-disulfonsäure ist ein wichtiges Zwischenprodukt bei der Herstellung von 4,4'-Dihydroxydiphenyl, welches zunehmend Bedeutung erlangt als Ausgangsmaterial für die Herstellung von hochtemperaturbeständigen Kondensationspolymeren. Das für die Polymerisation eingesetzte 4,4'-Dihydroxydiphenyl muß von hoher Reinheit sein. Dies setzt voraus, daß die Diphenyl-4,4'-disulfonsäure, aus der 4,4'-Dihydroxydiphenyl durch Alkalischmelze erhalten wird, selbst einen hohen Reinheitsgrad besitzt. Andernfalls ist ein hoher Reinigungsaufwand für das qualitativ schlechte Dihydroxydiphenyl, das aus einer Diphenyl-4,4'-disulfonsäure ungenügender Qualität resultiert, erforderlich.

Es ist bereits bekannt, Diphenyl-4,4'-disulfonsäure durch Sulfonierung von Diphenyl mit Schwefelsäure zu erhalten.

Ein solches Verfahren ist beispielsweise in der DE-OS 32 26 392 beschrieben. Es bedingt jedoch einen großen Schwefelsäure-Überschuß, wenn die Sulfonierungsreaktion annähernd quantitativ ablaufen soll, so daß erhebliche Mengen an Abfallsäure behandelt werden müssen. Ein weiterer Nachteil ist die umständliche Isolierung der Diphenyl-4,4'-disulfonsäure aus einer solchen Reaktionsmischung, die gemäß der DE-OS 32 26 392 nach Verdünnung mit Wasser durch Aussalzen als Monokaliumsalz der Disulfonsäure erfolgt. Für die Sulfonierungsreaktion und das Aussalzen sind nachteilig lange Zeiten erforderlich. Die genannte DE-OS 32 26 392 gibt als beste Ausbeute etwa 93 % der theoretischen Ausbeute an.

Es ist aus der DE-OS 18 04 693 auch bereits bekannt, Diphenyl mit Schwefeltrioxid zu sulfonieren. Bei diesem Verfahren werden etwa 2 Mol Schwefeltrioxid pro Mol Diphenyl in flüssigem Schwefeldioxid umgesetzt. Hierbei wird zuerst Diphenyl in flüssigem $SO_2$ gelöst und bei niedriger Temperatur, — 15 °C bis + 5 °C, $SO_3$ zu der Lösung zugefügt. Die Reaktionsmischung muß 2 bis 10 Stunden bei der genannten niedrigen Temperatur gehalten werden, da sonst beträchtliche Mengen an unerwünschten Isomeren und Sulfonen erzeugt werden. Danach wird die Temperatur auf mindestens + 10 °C erhöht und solange aufrechterhalten, bis die Bildung der Diphenyl-4,4'-disulfonsäure aufhört. Nach den Ausführungsbeispielen beträgt diese Weiterreaktionszeit bei 25 °C 2 Stunden. Die Isolierung der in $SO_2$ schwer löslichen Disulfonsäure erfolgt durch Filtration nach vorheriger Abkühlung auf unter — 10 °C. Das erhaltene Produkt wird bevorzugt mit flüssigem $SO_2$ auf dem Filter gewaschen. Von Nachteil bei diesem Verfahren ist die niedrige Mischungstemperatur. Um die bei der exothermen Sulfonierungsreaktion freiwerdende erhebliche Wärmemenge rasch abführen zu können, muß ein Kühlmedium bereitgestellt werden, dessen Temperatur deutlich unter der erlaubten Mischungstemperatur von — 15 °C bis + 5 °C liegen muß, was einen erheblichen technischen Aufwand bedeutet. Die lange Reaktionszeit von mindestens 4 Stunden nach dem Vermischen der Reaktionspartner ist ein weiterer gravierender Nachteil. Der Gehalt an Diphenyl-4,4'-disulfonsäure im Reaktionsgemisch beträgt in allen Ausführungsbeispielen der DE-OS 18 04 693 etwa 10 Gew.-% und ist deshalb unwirtschaftlich. Beim Arbeiten mit wirtschaftlicheren Gehalten von etwa 25-30 Gew.-% Disulfonsäure in der ausreagierten Reaktionsmischung steigen die Verunreinigungen in der isolierten Diphenyl-4,4'-disulfonsäure stark an. Das Produkt enthält dann etwa 10 Gew.-% Sulfone in Form von Sulfondisulfonsäuren (vgl. vorliegende Vergleichsbeispiele 5 und 6). Ein Produkt mit einem so hohen Sulfon-Gehalt ist jedoch für die Alkalischmelze zu 4,4'-Dihydroxydiphenyl, das für die genannten Kondensationspolymeren verwendet werden soll, nicht geeignet. Ein weiterer Nachteil des Verfahrens der DE-OS 18 04 693 ist die durch die Feinteiligkeit der Diphenyl-4,4'-disulfonsäure behinderte Filtration.

Während die DE-OS 18 04 693 eine vielstufige Temperatur- und Zeitführung für erforderlich hält und dennoch die geschilderten Nachteile nicht überwinden kann, wurde überraschend gefunden, daß durch eine besondere Art des Zusammengebens der Reaktionspartner.

a) die Bildung unerwünschter Nebenprodukte, wie Isomere und Sulfone, weitgehend unterdrückt wird ;

b) die gesamte Reaktionszeit wesentlich gesenkt wird ;

c) die Reaktion auch bei höherer Temperatur durchführbar ist, was wesentlich geringeren technischen Aufwand bei der Abführung der Reaktionswärme als bisher bedeutet ;

d) es möglich ist, bei wesentlich höheren und somit wirtschaftlicheren Gehalten an Diphenyl-4,4'-disulfonsäure im Reaktionsgemisch als bisher zu arbeiten, und

e) die erfindungsgemäß erhaltene Diphenyl-4,4'-disulfonsäure in hervorragend filtrierbarer und handhabbarer Form erhalten wird.

Es wurde also ein Verfahren zur Herstellung von Diphenyl-4,4'-disulfonsäure durch Sulfonierung von Diphenyl mit Schwefeltrioxid in Gegenwart eines inerten Lösungsmittels gefunden, das dadurch gekennzeichnet ist, daß man Schwefeltrioxid und Diphenyl bei einer Temperatur von — 30 °C bis + 60 °C so in den Reaktor eindosiert, daß während des Dosierens pro Mol Diphenyl 2-3 Mol $SO_3$ gleichzeitig mit dem Diphenyl zugegeben werden.

Es ist demnach erfindungswesentlich, daß während des Dosierens pro Mol in den Reaktor eindosierten Diphenyls 2-3 Mol $SO_3$, bevorzugt 2-

2,5 Mol $SO_3$, besonders bevorzugt 2,05-2,3 Mol $SO_3$, gleichzeitig mit dem Diphenyl zugegeben werden. Dies kann beispielsweise erreicht werden, indem man Schwefeltrioxid und Diphenyl in den angegebenen Molverhältnissen simultan in den Reaktor dosiert. Als simultanes Dosieren sei beispielsweise das Eintropfen von $SO_3$ und Diphenyl aus Tropftrichtern oder das Einpumpen der beiden Stoffe mit Hilfe üblicher Pumpen, wie Kreiselpumpen, Kolbenpumpen oder Membranpumpen genannt. Das $SO_3$ kann auch in geeigneter Weise gasförmig eindosiert werden. Im Reaktor sorgt man, beispielsweise durch Rühren, für eine intensive Vermischung der Reaktionspartner.

Die Temperatur bei der Zugabe und Vermischung der Reaktionspartner liegt erfindungsgemäß im Bereich von —30 °C bis + 60 °C, bevorzugt im Bereich von —15 °C bis + 50 °C, besonders bevorzugt bei 0-45 °C und ganz besonders bevorzugt bei 6-42 °C.

Die Dosierzeit der Reaktionspartner ist nicht kritisch. In der Praxis wird man zur Erhöhung der Raum-Zeit-Ausbeute die kürzeste Dosierzeit anstreben, die ihrerseits durch die Geschwindigkeit, mit der die Reaktionswärme abgeführt werden kann, bestimmt ist. Nach der Dosierung der Reaktionspartner kann eine Nachreaktionszeit zur Vervollständigung der Umsetzung angeschlossen werden. Diese Nachreaktionszeit kann wesentlich kürzer bemessen werden als nach Verfahren des Standes der Technik ; sie beträgt wenige Minuten bis etwa 1 Stunde in Abhängigkeit von der gewählten Vermischungstemperatur ; bei höherer Vermischungstemperatur wird die Nachreaktionszeit im allgemeinen kürzer. Die Temperatur bei der Nachreaktion liegt im allgemeinen oberhalb der Vermischungstemperatur aber noch innerhalb des Bereiches bis + 60 °C. Bewährt hat sich eine Verfahrensweise, bei der während der Vermischung der Reaktionspartner im Reaktionsraum eine Temperatur von 6-42 °C eingehalten wird und anschließend zur Nachreaktion die Temperatur auf 20-60 °C erhöht wird.

Als Lösungsmittel für das erfindungsgemäße Verfahren kommen alle Lösungsmittel in Betracht, die unter den Reaktionsbedingungen mit Schwefeltrioxid nicht reagieren und ein ausreichendes Lösungsvermögen für Schwefeltrioxid und Diphenyl aufweisen. Beispielhaft seien $SO_2$ oder chlorierte, aliphatische Kohlenwasserstoffe, bevorzugt die letzteren, genannt. Unter diesen wiederum seien die niedrigsiedenden, wie Methylenchlorid, 1,2-Dichlorethan und 1,2-Dichlorpropan, insbesondere Methylenchlorid, hervorgehoben.

Überraschenderweise wurde weiterhin gefunden, daß beim Arbeiten im oberen Teil des beanspruchten Temperaturbereiches das Produkt am Ende der Reaktion in Form von Granalien vorliegt. Der Granulierungseffekt tritt ab etwa 15 °C, stärker ab 20 °C, besonders deutlich ab 30 °C auf. Bei 40 °C fällt nahezu die gesamte Diphenyl-4,4'-disulfonsäure in Form von Granalien mit einem Durchmesser von 0,5-1 mm an. Die Granalienform ist von großem Vorteil, wenn die Diphenyl-4,4'-disulfonsäure beispielsweise in fester, lösungsmittelfreier Form gewünscht wird. Wegen der Größe und des hohen spezifischen Gewichtes sind die Granalien leicht durch Filtration oder Sedimentation aus der Reaktionsmischung abzutrennen und wegen ihres niedrigen Rest-Lösungsmittelgehaltes auch leicht zu trocknen. Diphenyl-4,4'-disulfonsäure in Form von Granalien ist rieselfähig und somit bei der Weiterverarbeitung leicht als Feststoff zu dosieren. Die Granalienform wirkt sich auch sehr vorteilhaft auf die Rührfähigkeit konzentrierter Suspensionen der Disulfonsäure in Lösungsmitteln aus und gestattet somit die erfindungsgemäße Durchführung der Sulfonierung bis zu wesentlich höheren Gehalten der Disulfonsäure im Reaktionsgemisch als bisher.

Das Lösungsmittel kann in der erfindungsgemäßen Reaktion auf verschiedene Weise eingesetzt werden, wobei man von einem oder mehreren der genannten Lösungsmittel, beispielsweise einem Gemisch, ausgehen kann. So kann man beide Reaktionspartner getrennt in einem oder mehreren Lösungsmitteln lösen und die Lösungen im Reaktionsgefäß vermischen. Weiterhin ist es möglich, nur einen der Reaktionspartner im Lösungsmittel zu lösen und den anderen in unverdünnter Form in das Reaktionsgefäß zum Vermischen einzubringen. Hierbei kann das Diphenyl in geschmolzener Form, das Schwefeltrioxid in flüssiger oder gasförmiger Form angewendet werden. Weiterhin kann ein Teil des Lösungsmittels im Reaktionsgefäß vorgelegt werden und die Reaktionspartner in einer der obengenannten Formen zudosiert werden. Die Vorlage eines Teils des Lösungsmittels, beispielsweise 5-60 % der gesamten Lösungsmittelmenge, bewährt sich besonders, wenn das $SO_3$ gasförmig eingesetzt wird.

Die Menge des benutzten Lösungsmittels ist nicht kritisch. Aus wirtschaftlichen Gründen wird man zum Auflösen der Reaktionspartner $SO_3$ und Diphenyl nicht wesentlich mehr Lösungsmittel verwenden, als zur Erreichung gesättigter Lösungen erforderlich ist. Wählt man beispielsweise eine Arbeitsweise, bei der die Reaktionspartner in Form von auf —10 °C gekühlten Lösungen im besonders bevorzugten Lösungsmittel Methylenchlorid eingesetzt werden sollen, so kann man die genannten Reaktionspartner in Form von etwa 20-25 gew.-%igen Lösungen einsetzen. Am Ende der Reaktion sollte in der Reaktionsmischung mindestens so viel Lösungsmittel vorliegen, daß die entstandene Suspension gut zu rühren bzw. zur Entleerung des Reaktionsgefäßes gut zu fördern ist. Im Falle von Methylenchlorid als Lösungsmittel hat sich erwiesen, daß eine Suspension mit einem Gehalt von 30-40 Gew.-% Diphenyl-4,4'-disulfonsäure nach beendeter Reaktion noch bequem zu rühren und zu fördern ist.

Der Einsatz niedrigsiedender Lösungsmittel erlaubt es ferner, während der Reaktion einen Teil des vorgelegten oder gemeinsam mit den Reaktionspartnern eingesetzten Lösungsmittels abzudestillieren.

Auf diese Weise können ursprünglich mit größerer Verdünnung angesetzte Reaktionsgemische zur rationelleren Aufarbeitung konzentriert werden. Weiterhin erlaubt das Abdestillieren des Lösungsmittels während der Reaktion, die exotherme Reaktionswärme aus dem Reaktionsgefäß abzuführen. Hierzu kann durch Anlegen eines geeigneten Unterdrucks der Siedepunkt des eingesetzten Lösungsmittels mit der gewünschten Reaktionstemperatur in Übereinstimmung gebracht werden. Dieser Abtransport der Reaktionswärme durch die sogenannte « Siedekühlung » ist jedoch auch bei konzentrierten Reaktionsmischungen möglich, wenn man durch einen geeignet tief gekühlten Rückflußkühler dafür sorgt, daß das verdampfende Lösungsmittel nach seiner Kondensation in die Reaktionsmischung zurückgeführt wird.

In einer besonderen Ausführungsform des erfindungsgemäßen Verfahrens hat sich gezeigt, daß durch das Vorlegen einer kleinen Menge des Reaktionsproduktes Diphenyl-4,4'-disulfonsäure vor Beginn des Zudosierens der Reaktionspartner eine vollständige Umsetzung dieser Reaktionspartner gefördert wird und gleichzeitig das unerwünschte Auftreten von Ablagerungen an den Reaktorwänden und am Rührer verhindert wird, wodurch eine wesentliche Verkürzung der Reaktionszeit erreicht wird. Die vorgelegte Menge an Diphenyl-4,4'-disulfonsäure beträgt 0,1-15 Mol, bevorzugt 1-10 Mol, besonders bevorzugt 3-7 Mol der Disulfonsäure pro 100 Mol umzusetzendes Diphenyl und wird im allgemeinen als Suspension in einem Teil von gleichzeitig vorgelegtem Lösungsmittel eingesetzt.

Im erfindungsgemäßen Verfahren wird eine sehr niedrige Sulfonbildung erzielt, die unter 1,5 Mol-%, bezogen auf die Molzahl des eingesetzten Diphenyls, liegt. In einer besonderen Ausführungsform wird zur nahezu vollständigen Unterdrückung der Sulfonbildung in Gegenwart eines Sulfoninhibitors gearbeitet, der in einer Menge von 0,1 bis zu 10 Gew.-%, bevorzugt in einer Menge von 2-5 Gew.-%, bezogen auf das einzusetzende Diphenyl, angewandt wird. Als Sulfoninhibitor können beispielsweise Carbonsäuren oder deren Anhydride, wie Essigsäure, Propionsäure oder Essigsäureanhydrid, Percarbonsäuren, wie Peressigsäure oder Perpropionsäure, Pyridinverbindungen, Dimethylformamid oder Salze, wie Natriumsulfat, eingesetzt werden. In bevorzugter Weise wird Essigsäure oder Essigsäureanhydrid eingesetzt. Sulfoninhibitoren sind in der Literatur bei der Umsetzung anderer Substrate als Diphenyl bereits beschrieben (E.E. Gilbert, Sulfonation and Related Reactions, Interscience Publishers, New York 1965, Seite 66 ff ; R. Fleury, Sulfonierung von Aromaten mit $SO_3$, Dissertation ETH Zürich, 1966, Kapitel 4).

Der Sulfoninhibitor kann auf verschiedene Weise in das Reaktionsgemisch eingebracht werden. So kann der Inhibitor vor dem Eindosieren der Reaktionspartner im Reaktionsgefäß vorgelegt werden ; er kann jedoch auch während des Zudosierens der Reaktionspartner getrennt oder gemeinsam mit einem oder beiden der Reaktionspartner eingebracht werden. Es ist weiterhin möglich, Teilmengen des Inhibitors gleichzeitig in den verschiedenen angegebenen Weisen in das Reaktionsgemisch einzubringen. In bevorzugter Weise wird die gesamte Menge des Inhibitors im Reaktionsgefäß vor der Zugabe der Reaktionspartner vorgelegt.

Während der Durchführung des erfindungsgemäßen Verfahrens ist für eine ausreichende Durchmischung der eindosierten Reaktionspartner, beispielsweise durch intensives Rühren, zu sorgen. Nach beendeter Reaktion liegt eine gut rührbare Suspension von körniger Diphenyl-4,4'-disulfonsäure in dem gewählten Lösungsmittel vor. Die Aufarbeitung des Reaktionsgemisches und die Isolierung der Disulfonsäure kann auf verschiedene Weise erfolgen :

a) Infolge der ausgezeichneten Filtrierbarkeit der Diphenyl-4,4'-disulfonsäure ist deren Abtrennung aus dem Reaktionsgemisch durch Filtration eine einfach durchführbare Aufarbeitungsform. Die Mutterlauge kann für eine nachfolgende Sulfonierung wieder eingesetzt werden. Der Filterrückstand ergibt sodann nach Trocknung, gegebenenfalls im Vakuum unter Erwärmen, die Disulfonsäure in lösungsmittelfreier Form. Auch durch Eindampfen des Reaktionsgemisches kommt man zu einer lösungsmittelfreien Disulfonsäure.

b) Beim Einsatz eines Chloralkans oder eines anderen wasserunlöslichen Lösungsmittels kann man das Reaktionsgemisch auch mit Wasser extrahieren und so beispielsweise bei 40 °C eine etwa 50 gew.-%ige wäßrige Lösung der Disulfonsäure erhalten.

c) Es ist auch möglich, das ausreagierte Reaktionsgemisch auf wenig Wasser zu geben und dann das wasserunlösliche Lösungsmittel abzudestillieren, so daß am Ende im Sumpf beispielsweise bei 70 °C eine etwa 70-75 gew.-%ige geschmolzene wasserhaltige Diphenyl-4,4'-disulfonsäure vorliegt. Eine solche wasserhaltige Disulfonsäure kann beispielsweise direkt mit Natronlauge nach dem Verfahren der EP-Anmeldung 85 883 zu sehr reinem 4,4'-Dihydroxydiphenyl umgesetzt werden.

In allen genannten Ausführungsformen der Sulfierungsreaktion und der Aufarbeitung wird die Diphenyl-4,4'-disulfonsäure in hoher Ausbeute und in sehr reiner Form nach einem äußerst wirtschaftlich durchzuführenden Verfahren erhalten. Die erfindungsgemäß erhaltene Diphenyl-4,4'-disulfonsäure kann ferner in granulierter Form erhalten werden, wodurch die Abtrennung vereinfacht und die Handhabung bei der Weiterverwendung wesentlich erleichtert wird, während nach Verfahren des Standes der Technik sehr feinteilige, schlecht filtrierbare Produkte anfallen. Die Diphenyl-4,4'-disulfonsäure enthält im allgemeinen nur sehr geringe Mengen an Diphenylmonosulfonsäure (höchstens 0,2 Gew.-%) und an isomeren Disulfonsäuren (höchstens 1 Gew.-

%) und ist praktisch frei von Sulfonen (weniger als 1 Gew.-%).

Beispiel 1

16 g (0,05 Mol) Diphenyl-4,4'-disulfonsäure und 3,0 g (0,05 Mol) Essigsäure wurden in 500 ml Methylenchlorid vorgelegt. In diese Vorlage wurde bei + 6 °C innerhalb 1 1/4 h unter Rühren eine Lösung von 154 g (1,00 Mol) Diphenyl in 400 ml Methylenchlorid eingetropft und gleichzeitig 178 g (2,22 Mol) Schwefeltrioxid durch ein Rohr gasförmig unter die Flüssigkeitsoberfläche eingeführt.

Man rührte 1/4 h bei + 6 °C nach, erwärmte auf Raumtemperatur und rührte noch 1 h nach.

Die Kristalle wurden abgesaugt, mit Methylenchlorid gewaschen und bei 40 °C im Vakuum getrocknet ; Ausbeute : 348 g 92,5 %ige Diphenyl-4,4'-disulfonsäure ≙ 97,6 % der theoretischen Ausbeute.

Das erhaltene Produkt enthielt :
Diphenyl-4,4'-disulfonsäure : 92,5 Gew.-%
isomere Diphenyldisulfonsäuren : ca. 1 Gew.-%
Diphenyl-4-sulfonsäure : 0,2 Gew.-%
Sulfon-disulfonsäuren : 0,9 Gew.-%.

Beispiel 2

16 g (0,05 Mol) Diphenyl-4,4'-disulfonsäure und 3,0 g (0,05 Mol) Essigsäure wurden in 200 ml Methylenchlorid vorgelegt. In diese Vorlage wurden bei + 20 °C unter Rühren innerhalb ca. 1 1/4 h gleichzeitig 154 g (1,00 Mol) Diphenyl in Form einer etwa 26 %igen Lösung in Methylenchlorid und 176 g (2,20 Mol) Schwefeltrioxid in Form einer ca. 22 %igen, auf — 10 °C gekühlten Lösung in Methylenchlorid getropft.

Man rührte 1/4 h bei + 20 °C nach, erwärmte auf 40 °C und rührte noch 1 h nach.

Die Reaktionsmischung gab man auf eine Vorlage von 220 ml Wasser (exotherme Reaktion) und destillierte das gesamte Methylenchlorid ab. Anschließend destillierte man bei schwachem Vakuum bei einer Sumpftemperatur von 70 °C noch etwa 100 ml Wasser ab und erhielt 441 g wasserfeuchte 72,9 %ige Diphenyl-4,4'-disulfonsäure in bei 70 °C flüssiger Form ; Ausb. 97,5 % der theoretischen Ausbeute.

Das erhaltene Produkt enthielt :
Diphenyl-4,4'-disulfonsäure : 72,9 Gew.-%
isomere Diphenyldisulfonsäuren : 0,7 Gew.-%
Diphenyl-4-sulfonsäure : 0,2 Gew.-%
Sulfon-disulfonsäuren : 0,7 Gew.-%.

Beispiel 3

32 g (0,1 Mol) Diphenyl-4,4'-disulfonsäure und 6,0 g (0,1 Mol) Essigsäure wurden in 200 ml Methylenchlorid vorgelegt. Man evakuierte das Reaktionsgefäß so, daß die Vorlage bei + 10 °C siedete (ca. 300 mbar). In die gerührte, siedende Vorlage wurden bei + 10 °C innerhalb etwa 2 h gleichzeitig 231 g (1,50 Mol) Diphenyl und 276 g (3,45 Mol) Schwefeltrioxid, jeweils in Form von auf — 10 °C gekühlten, 20 %igen Lösungen in Methylenchlorid, eindosiert. Gleichzeitig ließ man 950 ml Methylenchlorid abdestillieren.

Die etwa 35 %ige Suspension von Diphenyl-4,4'-disulfonsäure rührte man 1/4 h bei + 10 °C nach, erwärmte auf 40 °C und rührte bei 40 °C 1 h nach.

Die Kristalle wurden abgesaugt, mit Methylenchlorid gewaschen und bei 40 °C im Vakuum getrocknet ; Ausbeute : 545 g 89 %ige Diphenyl-4,4'-disulfonsäure ≙ 96,5 % der theoretischen Ausbeute.

Das erhaltene Produkt enthielt :
Diphenyl-4,4'-disulfonsäure : 89 Gew.-%
isomere Diphenyldisulfonsäuren : ca. 1 Gew.-%
Diphenyl-4-sulfonsäure : 0,2 Gew.-%
Sulfon-disulfonsäuren : 0,8 Gew.-%.

Beispiel 4

16 g (0,05 Mol) Diphenyl-4,4'-disulfonsäure und 3 g (0,05 Mol) Essigsäure wurden in 200 ml Methylenchlorid vorgelegt und zum Sieden erhitzt (etwa 40 °C). Sodann wurden unter Rühren innerhalb etwa 1 h gleichzeitig 154 g (1 Mol) Diphenyl in Form einer etwa 26 gew.-%igen Lösung in Methylenchlorid und 176 g (2,2 Mol) Schwefeltrioxid in Form einer etwa 22 gew.-%igen, auf — 10 °C gekühlten Lösung in Methylenchlorid zugetropft. Man rührte 1 h bei + 40 °C nach. Die in Form von Granalien angefallene Diphenyl-4,4'-disulfonsäure wurde bei Raumtemperatur abfiltriert, mit Methylenchlorid gewaschen und bei + 40 °C im Vakuum getrocknet. Ausbeute : 348 g 92,4 %ige Diphenyl-4,4'-disulfonsäure, entsprechend 97,4 % der theoretischen Ausbeute.

Das erhaltene Produkt enthielt :
Diphenyl-4,4'-disulfonsäure : 92,4 Gew.-%
isomere Diphenyldisulfonsäuren : ca. 1 Gew.-%
Diphenyl-4-sulfonsäure : 0,2 Gew.-%
Sulfon-disulfonsäuren : 0,9 Gew.-%.

Beispiel 5 (Vergleichsbeispiel)

Bis auf die höhere Konzentration an vorgelegter Diphenyllösung wurde gemäß Beispiel 1 der DE-OS 18 04 693 verfahren. In einem Autoklaven löste man hierzu 92,4 g (0,6 Mol) Diphenyl in 570 g Schwefeldioxid (13,9 gew.-%ige Lösung ; die DE-OS 18 04 693 benutzt eine 5,1 gew.-%ige Lösung) und tropfte unter Rühren bei — 10 °C innerhalb 1,5 h 101 g (1,26 Mol) flüssiges Schwefeltrioxid dazu. Man rührte 3 h bei — 10 °C nach, erhöhte die Temperatur auf + 25 °C und hielt 2 h bei + 25 °C. Nach Abkühlen auf unter — 10 °C filtrierte man die feinpulverige Diphenyl-4,4'-disulfonsäure ab und wusch den Niederschlag auf der Nutsche mit flüssigem Schwefeldioxid. Nach Trocknung bei + 40 °C im Vakuum wurden 188 g 85,8 %ige Diphenyl-4,4'-disulfonsäure erhalten, entsprechend einer Ausbeute von 85,6 % der theoretischen Ausbeute.

Das erhaltene Produkt enthielt :
Diphenyl-4,4'-disulfonsäure : 85,8 Gew.-%

isomere Diphenyldisulfonsäuren : ca. 1 Gew.-%
Diphenyl-4-sulfonsäure : 0,3 Gew.-%
Sulfon-disulfonsäuren : 11 Gew.-%.

Beispiel 6 (Vergleichsbeispiel)

Die Sulfonierung von Beispiel 5 wurde mit dem Unterschied wiederholt, daß mit Schwefeldioxid verdünntes Schwefeltrioxid eingesetzt wurde. In einem Autoklaven mit Rückflußkühler rührte man eine bei — 10 °C siedende Lösung von 92,4 g (0,6 Mol) Diphenyl in 570 g Schwefeldioxid und tropfte durch den Rückflußkühler innerhalb von 1 1/2 h 101 g (1,26 Mol) Schwefeltrioxid dazu. Der Rückfluß des Schwefeldioxids wurde so bemessen, daß nach der im Rückflußkühler stattfindenden Vermischung von Schwefeltrioxid und Schwefeldioxid eine ca. 10 gew.-%ige Lösung des $SO_3$ in $SO_2$ in die vorgelegte Diphenyllösung tropfte. Die weitere Reaktionsführung und die Aufarbeitung erfolgten, wie in Beispiel 5 beschrieben. Es wurden 189 g 86,8 %ige Diphenyl-4,4'-disulfonsäure erhalten, entsprechend einer Ausbeute von 87,1 % der theoretischen Ausbeute.
Das erhaltene Produkt enthielt :
Diphenyl-4,4'-disulfonsäure : 86,8 Gew.-%
isomere Diphenyldisulfonsäuren : ca. 1 Gew.-%
Diphenyl-4-sulfonsäure : 0,3 Gew.-%
Sulfon-disulfonsäuren : 10 Gew.-%.

**Patentansprüche**

1. Verfahren zur Herstellung von Diphenyl-4,4'-disulfonsäure durch Sulfonierung von Diphenyl mit Schwefeltrioxid in Gegenwart eines inerten Lösungsmittels, dadurch gekennzeichnet, daß man Schwefeltrioxid und Diphenyl bei einer Temperatur von — 30 °C bis + 60 °C so in den Reaktor eindosiert, daß während des Dosierens pro Mol Diphenyl 2-3 Mol $SO_3$ gleichzeitig mit dem Diphenyl zugegeben werden.

2. Verfahren nach Anspruch 1, dadurch gekennzeichnet, daß man die Zugabe von Diphenyl und Schwefeltrioxid bei einer Temperatur von — 15 °C bis + 50 °C vornimmt.

3. Verfahren nach Anspruch 1 und 2, dadurch gekennzeichnet, daß während des Dosierens pro Mol Diphenyl 2-2,5 Mol Schwefeltrioxid gleichzeitig mit dem Diphenyl zugegeben werden.

4. Verfahren nach Anspruch 1 bis 3, dadurch gekennzeichnet, daß man als Lösungsmittel Chloralkane einsetzt.

5. Verfahren nach Anspruch 1 bis 4, dadurch gekennzeichnet, daß man als Lösungsmittel Methylenchlorid einsetzt.

6. Verfahren nach Anspruch 1 bis 5, dadurch gekennzeichnet, daß Diphenyl als Lösung und Schwefeltrioxid gasförmig in den Reaktor eindosiert werden.

7. Verfahren nach Anspruch 1 bis 6, dadurch gekennzeichnet, daß in Gegenwart eines Sulfoninhibitors in einer Menge von 0,1 bis 10 Gew.-%, bezogen auf einzusetzendes Diphenyl, gearbeitet wird.

8. Verfahren nach Anspruch 1 bis 7, dadurch gekennzeichnet, daß als Sulfoninhibitor Essigsäure oder Essigsäureanhydrid eingesetzt wird.

9. Verfahren nach Anspruch 1 bis 8, dadurch gekennzeichnet, daß vor Beginn der Zugabe von Schwefeltrioxid und Diphenyl pro 100 Mol Diphenyl-Einsatz 0,1-15 Mol Diphenyl-4,4'-disulfonsäure als Suspension im Lösungsmittel im Reaktor vorgelegt werden.

10. Verfahren nach Anspruch 1 bis 9, dadurch gekennzeichnet, daß man einen Teil des Lösungsmittels, den Sulfoninhibitor und 0,1-15 Mol Diphenyl-4,4'-disulfonsäure pro 100 Mol Diphenyl-Einsatz vorlegt und dann Diphenyl als Lösung und $SO_3$ als Lösung oder gasförmig zudosiert.

**Claims**

1. Process for preparing diphenyl-4,4'-disulphonic acid by sulphonating diphenyl with sulphur trioxide in the presence of an inert solvent, characterised in that sulphur trioxide and diphenyl are metered into the reactor at a temperature of — 30 °C to + 60 °C in such a way that in the course of the metering 2-3 moles of $SO_3$ are added per mole of diphenyl at the same time as the diphenyl.

2. Process according to Claim 1, characterised in that the addition of diphenyl and sulphur trioxide is carried out at a temperature of — 15 °C to + 50 °C.

3. Process according to Claims 1 and 2, characterised in that, during the metering, 2-2.5 moles of sulphur trioxide are added per mole of diphenyl at the same time as the diphenyl.

4. Process according to Claims 1 to 3, characterised in that chloroalkanes are used as the solvent.

5. Process according to Claims 1 to 4, characterised in that methylene chloride is used as the solvent.

6. Process according to Claims 1 to 5, characterised in that diphenyl is metered into the reactor in the form of a solution and sulphur trioxide in the form of a gas.

7. Process according to Claims 1 to 6, characterised in that the reaction is carried out in the presence of a sulphone-inhibitor in an amount of 0.1 to 10 % by weight, relative to diphenyl to be used.

8. Process according to Claims 1 to 7, characterised in that acetic acid or acetic anhydride is used as the sulphone-inhibitor.

9. Process according to Claims 1 to 8, characterised in that before the start of the addition of sulphur trioxide and diphenyl 0.1-15 moles of diphenyl-4,4'-disulphonic acid per 100 moles of diphenyl used are introduced into the reactor in the form of a suspension in the solvent.

10. Process according to Claims 1 to 9, characterised in that a portion of the solvent, the sulphone-inhibitor and 0.1-15 moles of diphenyl-4,4'-disulphonic acid per 100 moles of diphenyl used are initially introduced and then diphenyl, in the form of a solution, and SO₃, in the form of a solution or in the form of a gas, are metered in.

**Revendications**

1. Procédé de production d'acide diphényl-4,4'-disulfonique par sulfonation de diphényle avec de l'anhydride sulfurique en présence d'un solvant inerte, caractérisé en ce qu'on injecte de l'anhydride sulfurique et du diphényle dans le réacteur à une température de — 30 °C à + 60 °C de façon telle que pendant l'injection, on ajoute par mole de diphényle 2 à 3 moles de SO₃ en même temps que le diphényle.

2. Procédé suivant la revendication 1, caractérisé en ce que l'addition de diphényle et d'anhydride sulfurique est effectuée à une température de — 15 à + 50 °C.

3. Procédé suivant les revendications 1 et 2, caractérisé en ce que pendant l'injection, on ajoute par mole de diphényle 2 à 2,5 moles d'anhydride sulfurique en même temps que le diphényle.

4. Procédé suivant les revendications 1 à 3, caractérisé en ce qu'on utilise des chloralcanes comme solvants.

5. Procédé suivant les revendications 1 à 4, caractérisé en ce qu'on utilise du chlorure de méthylène comme solvant.

6. Procédé suivant les revendications 1 à 5, caractérisé en ce que le diphényle est injecté sous forme de solution et l'anhydride sulfurique est injecté sous la forme gazeuse dans le réacteur.

7. Procédé suivant les revendications 1 à 6, caractérisé en ce qu'on opère en présence d'un inhibiteur de sulfone en une quantité de 0,1 à 10 % en poids par rapport au diphényle devant être utilisé.

8. Procédé suivant les revendications 1 à 7, caractérisé en ce qu'on utilise comme inhibiteur de sulfone l'acide acétique ou l'anhydride d'acide acétique.

9. Procédé suivant les revendications 1 à 8, caractérisé en ce qu'on introduit préalablement dans le réacteur avant le début de l'addition d'anhydride sulfurique et de diphényle, pour 100 moles de charge de diphényle, de 0,1 à 15 moles d'acide diphényl-4,4'-disulfonique en suspension dans un solvant.

10. Procédé suivant les revendications 1 à 9, caractérisé en ce qu'on introduit préalablement une partie du solvant, l'inhibiteur de sulfone et 0,1 à 15 moles d'acide diphényl-4,4'-disulfonique pour 100 moles de charge de diphényle, puis on ajoute du diphényle sous forme de solution et du SO₃ sous forme de solution ou sous la forme gazeuse.